# EUROPEAN PATENT SPECIFICATION

(11) **EP 2 805 166 B1**
(45) Date of publication and mention of the grant of the patent: **21.11.2018**
(21) Application number: 13701421.3
(22) Date of filing: 21.01.2013
(51) Int. Cl.: G01N 33/574

(54) **METHOD FOR THE PROGNOSIS OF SURVIVAL TIME OF A PATIENT SUFFERING FROM A SOLID CANCER**
VERFAHREN ZUR VORHERSAGE DER ÜBERLEBENSZEIT EINES PATIENTEN, DER AN KREBS LEIDET
PROCÉDÉ DE PRONOSTIC DE LA DURÉE DE SURVIE D'UN PATIENT SOUFFRANT D'UN CANCER SOLIDE

(30) Priority: 20.01.2012 EP 12151875
(43) Date of publication of application: 26.11.2014
(73) Proprietor: INSERM - Institut National de la Santé et de la Recherche Médicale, 75013 Paris (FR); Université Paris VII (Denis Diderot), 75013 Paris (FR); Université Paris Descartes - Paris V, 75006 Paris (FR); Assistance Publique - Hôpitaux de Paris, 75004 Paris (FR); Sorbonne Université, 75006 Paris (FR)
(72) Inventor: DIEU-NOSJEAN, Marie-Caroline, F-75270 Paris Cedex 06 (FR); FRIDMAN, Wolf Hervé, 15 rue de l'Ecole de Médecine F-75006 Paris (FR); REMARK, Romain, New York, NY 10029 (US); SAUTES-FRIDMAN, Catherine, 15 rue de l'Ecole de Médecine F-75006 Paris (FR)
(74) Representative: Cabinet Plasseraud
(86) International application number: PCT/EP2013/051047
(87) International publication number: WO 2013/107907

(56) References cited:
- EP-A1- 1 777 523
- ANDREA LADÃ NYI ET AL: "Prognostic impact of B-cell density in cutaneous melanoma", CANCER IMMUNOLOGY, IMMUNOTHERAPY, SPRINGER, BERLIN, DE, vol. 60, no. 12, 21 July 2011 (2011-07-21) , pages 1729-1738, XP019980939, ISSN: 1432-0851, DOI: 10.1007/S00262-011-1071-X
- NELSON BRAD H: "CD20(+) B Cells: The Other Tumor-Infiltrating Lymphocytes", JOURNAL OF IMMUNOLOGY, vol. 185, no. 9, November 2010 (2010-11), pages 4977-4982, XP002675333, ISSN: 0022-1767
- CATHERINE SAUTÃS-FRIDMAN ET AL: "Tumor microenvironment is multifaceted", CANCER AND METASTASIS REVIEWS, KLUWER ACADEMIC PUBLISHERS, DO, vol. 30, no. 1, 28 January 2011 (2011-01-28), pages 13-25, XP019885790, ISSN: 1573-7233, DOI: 10.1007/S10555-011-9279-Y
- FRIDMAN WOLF H ET AL: "Immune infiltration in human cancer: prognostic significance and disease control", CURRENT TOPICS IN MICROBIOLOGY AND IMMUNOLOGY, SPRINGER, BERLIN, DE, vol. 344, 1 January 2011 (2011-01-01), pages 1-24, XP009158913, ISSN: 0070-217X
- F PAGÈS ET AL: "Immune infiltration in human tumors: a prognostic factor that should not be ignored", ONCOGENE, vol. 29, no. 8, 25 February 2010 (2010-02-25), pages 1093-1102, XP055025840, ISSN: 0950-9232, DOI: 10.1038/onc.2009.416
- DIEU-NOSJEAN MARIE-CAROLINE ET AL: "Long-term survival for patients with non-small-cell lung cancer with intratumoral lymphoid structures.", JOURNAL OF CLINICAL ONCOLOGY : OFFICIAL JOURNAL OF THE AMERICAN SOCIETY OF CLINICAL ONCOLOGY 20 SEP 2008 LNKD- PUBMED:18802153, vol. 26, no. 27, 20 September 2008 (2008-09-20), pages 4410-4417, XP002675334, ISSN: 1527-7755

## Description

### FIELD OF THE INVENTION

The present invention relates to an *in vitro* method for the prognosis of survival time of a patient suffering from a solid cancer.

### BACKGROUND OF THE INVENTION

As indicated in Dieu-Nosjean et al. (J Clin Oncol 26:4410-4417. 2008), lung cancer is the most common cause of cancer related death in the world. Approximately 80% to 90% of cases involve Non-Small-Cell Lung Cancer (NSCLC), which includes adenocarcinoma and squamous cell carcinoma. Only patients whose tumors can be completely resected have a significant chance of increased survival. However, as many as 30% of patients with stage I disease experience recurrence after surgery. The correlation between tumor-infiltrating immune cells and the prognosis of patients with lung cancer is controversial.

A tumor is composed of malignant, stromal, endothelial, and immune cells that form a heterogeneous network and exhibit complex interactions. Although tumor eradication by the immune system is often inefficient, there is evidence that many developing cancers are not ignored by the immune system. Spontaneous tumor regressions occurring concomitantly with autoimmune manifestations and the higher incidence of tumors in immunosuppressed patients are indications of the involvement of the immune system in tumor rejection. Mice deficient in immune functions spontaneously develop tumors. The density of tumor-infiltrating lymphocytes (TILs) with cytotoxic and memory phenotypes is highly predictive of good clinical outcome. However, although prognosis is related to the homing of effector immune cells, it is still unclear where the activation of the specific immune response takes place: in the tumor, the draining lymph node, or both.
It is now well established that immune responses can take place at distance of secondary lymphoid organs, in tertiary lymphoid structures (TLS). Dieu-Nosjean et al. have observed that these lymph node-like structures can develop in lung cancer patients. They have been named "Tumor-induced Bronchus-Associated Lymphoid Tissues" (Ti-BALT) as they were never found in the non-tumoral tissues of NSCLC patients. Moreover, Dieu-Nosjean et al. have demonstrated that the density of mature DC, a population which was selectively detected in Ti-BALT, is associated with a favorable clinical outcome, suggesting that they represent an activation site for tumor-specific T cells.

The presence of TLS has been reported in other human tumors (e.g., colorectal and breast (Gobert et al., Cancer Res 2009; 69(5) 2000-2009) indicating that ectopic lymphoid structures arise in many solid tumors.

Ladanyi et al (Cancer Immunol Immunother. 2011 Dec;60(12):1729-38) disclose the prognostic impact of B-cell density in cutaneous melanoma.
Nelson (J Immunol. 2010 Nov 1;185(9):4977-82) is a review comparing B cell responses in cancer, autoimmunity and transplantation, with the goal of elucidating the mechanisms used by B cells to facilitate long-term T cell responses.
European patent application published under reference EP 1 777 523 discloses methods for determining the prognosis of a patient suffering from cancer by quantifying, in a tumor sample, at least one biological marker which is indicative of the status of the adaptive immune response of said patient against cancer.
Sautès-Fridman et al (Cancer Metastasis Rev. 2011 Mar;30(1):13-25) the influence of infectious context and immune cell infiltration organization in human Non-Small Cell Lung Cancers (NSCLC) progression.
Fridman et al (Curr Top Microbiol Immunol. 2011;344:1-24) and Pages et al (Oncogene. 2010 Feb 25;29(8):1093-102) discuss the prognostic significance of immune infiltration in human cancer.
Dieu-Nosjean et al (J Clin Oncol. 2008 Sep 20;26(27):4410-7) disclose that the number of tumor-infiltrating mature DCs may allow identifying patients with early-stage NSCLC who have a high risk of relapse.

### SUMMARY OF THE INVENTION

The present invention relates to an *in vitro* method for the prognosis of the survival time of a patient suffering from a solid cancer, comprising the quantification of the cell density of follicular B cells present in tumor-induced lymphoid structures from said patient, wherein a high density of follicular B cells indicates that the patient has a favorable prognosis and a low density of follicular B cells indicates that the patient has a poor prognosis.

The present invention provides a novel method for the prognosis of the survival time of solid cancer patients. The method is of higher accuracy than currently used staging methods (e.g. UICC-TNM) and thus fulfils a long-felt and ongoing need in the art to correctly and accurately predict the likely course or outcome of cancer in a patient, as reflected in survival time. The ability to do so enables medical practitioners to individually adapt cancer treatment protocols to particular patients. Patients who, according to the present method, have a high probability of a good therapy outcome may not need to receive the most aggressive treatments in order to experience a favorable outcome, and thus can avoid or minimize the side effects associated with such treatments, whereas patients with a poor prognosis can be treated aggressively at the earliest possible stage of the disease or by another therapy than the one used.

### DETAILED DESCRIPTION OF THE INVENTION

The present invention relates to an *in vitro* method for the prognosis of survival time of a patient suffering from a solid cancer, comprising the following steps:
a) quantifying in a tumor-induced lymphoid structure, the cell density of follicular B cells, and
b) comparing the cell density of follicular B cells value obtained at step a) with a predetermined reference value; and
c) providing a favorable prognosis of survival time for said patient when the cell density of follicular B cells is higher than said predetermined reference value, or providing a poor prognosis of survival time for said patient when the cell density of follicular B cells is lower than said predetermined reference value.

By tumor-induced lymphoid structure, it is meant the organization of tumor-infiltrating leukocytes into lymph-node like structure in the stroma of the tumor mass and, is composed of mature dendritic cell-T cell clusters (T-cell areas) and B-cell follicles (B-cell areas). Typically, depending on the tumor section, only one out of the two areas or both areas can be observed.

By follicular B cells, it is meant B cell subsets clustered into B-cell follicle. They are mainly of naive and germinal center phenotype.

The survival can be the disease-specific survival (DSS), the disease-free survival (DFS) or the overall survival (OS).

The present invention enables the evaluation of the risk of recurrence of a patient who has been surgically treated and, if needed, subsequently received the appropriate treatment (such as radiotherapy, chemotherapy and/or hormonal therapy).

The method of prognosis according to the invention may be used alone or in combination with any other methods already used for the prognostic assessment of solid cancers, including stage, demographic and anthropometric parameters, results of routine clinical or laboratory examination, including size of the tumor, histoprognostic grading, hormone receptors, oncotype ...

In a preferred embodiment, the method of the invention further comprises the steps of:
a) quantifying on the whole tumor, the cell density of mature dendritic cells, and
b) comparing the cell density of mature dendritic cells value obtained at step a) with a predetermined reference value; and
c) providing a favorable prognosis of survival time for said patient when the cell densities of both the follicular B cells and of the mature dendritic cells are higher than said predetermined reference values,
providing a poor prognosis of survival time for said patient when the cell densities of both follicular B cells and of the mature dendritic cells are lower than said predetermined reference values, or
providing an intermediate prognosis of survival time for said patient when one value of the cell densities of follicular B cells and of the mature dendritic cells is lower than said predetermined reference value and the other value is higher than said other predetermined reference value.

By mature dendritic cells, it is meant a population of dendritic cells that are professional for the presentation of processed antigens to T cells. Mature dendritic cells infiltrating the tumor are selectively located in contact with T cells, in the T-cell rich areas of the tumor-induced lymphoid structure.

Typically follicular B cells density and the mature dendritic cells density may be measured for example by immunohistochemistry performed on a tumor sections (frozen or paraffin-embedded tissue sections) of sample obtained by biopsy.

In an embodiment of the invention, total B cells are detected by immunohistochemistry with an antibody against the CD20 molecule. Among total B cells, follicular B cells are selectively counted on the whole tumor section.
In an embodiment of the invention, mature dendritic cells are detected by immunohistochemistry with an antibody against the DC-Lamp (CD208) molecule. Mature dendritic cells are counted on the whole tumor section. The density of cells may be expressed as the number of cells that are counted per one unit of surface area of the tumor section, e.g. as the number of cells that are counted per intermediate-power field (original magnification x100) or mm² of surface area of the tumor.

As the follicular B cells are organized into a cell aggregate in the B-cell follicle and as they represent more than 98% of total cells present in the B-cell follicle, the density of follicular B cells can also be measured as a total surface of B-cell follicles per one unit of surface area of the tumor, e.g. as the surface area of B-cell follicles in mm² per intermediate-power field (original magnification x100) or mm² of surface area of the tumor.

Typically, the predetermined reference values for the cell density of follicular B cells and for the cell density of mature dendritic cells may be determined by applying statistical methods in large-scale studies on cancer patients.

Examples of solid cancers with tumor-induced lymphoid structures are lung cancers, colorectal cancers and breast cancers.
In a preferred embodiment, the solid cancer is a lung cancer.
In a more preferred embodiment, the solid cancer is a non-small cell lung cancer.

In an embodiment of the invention, the patient is a patient with an early stage of cancer, such as stage I cancer.

In an alternative embodiment of the invention, the patient is an operable patient with advanced stage of cancer (up to stage IIIb cancer).

The stages of breast cancer and colo-rectal cancer are defined for example in UICC. TNM Classification of Malignant Tumours. 6th ed. Sobin LH, Wittekind Ch (eds) Wiley-Liss: New York, 2002.

In an embodiment of the invention, the patient is a patient with early stage of cancer who did not receive any neo-adjuvant, nor adjuvant therapy, such as chemotherapy and/or radiotherapy.

In an alternative embodiment of the invention, the patient is a patient with advanced stage of cancer who receives adjuvant therapy with or without neo-adjuvant therapy, such as chemotherapy and/or radiotherapy.

In the following, the invention will be illustrated by means of the following examples as well as the tables and figures.

### FIGURE LEGENDS

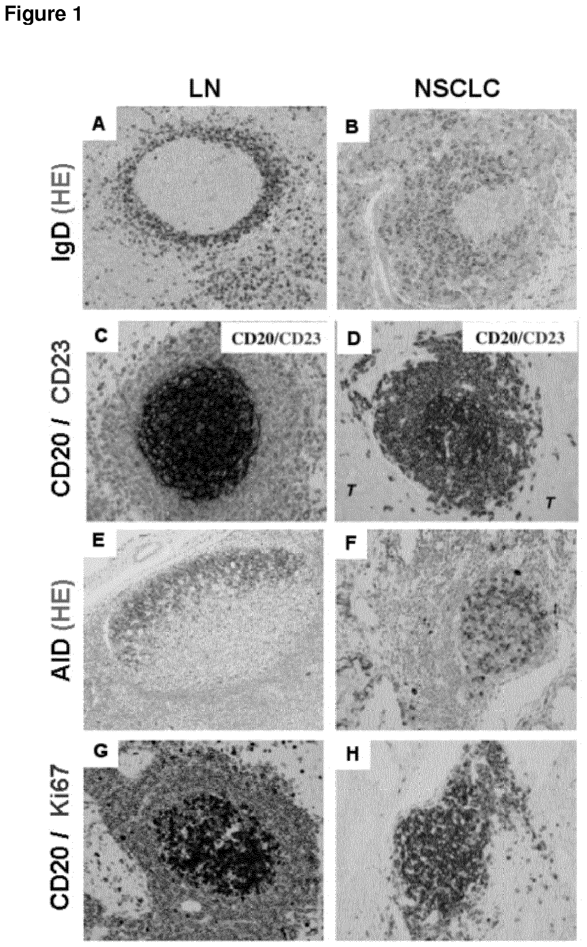

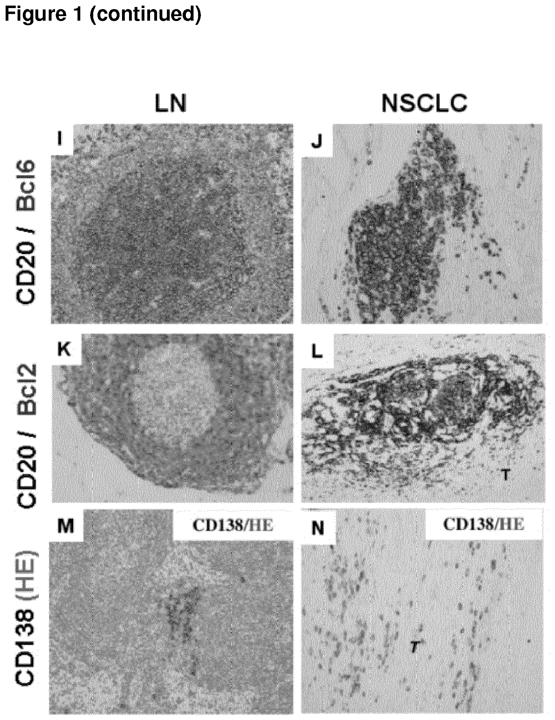

**Figure 1****. Characterization of B cell subsets into and outside Ti-BALT**
   Single (A-B, E-F, M-N) and double (C-D, G-L) immunostainings on paraffin-embedded lymph node (left column) and lung tumor (right column) sections. Ti-BALT B-cell areas present features of secondary follicles of reactive lymph nodes, as they are composed by: a mantle of IgD+ naive B cells (dark grey, B) surrounding a germinal centre. As in lymph nodes, CD20+ germinal centre B cells (dark grey, D, H, J, L) express the CD23 (black, D), AID (dark grey, F), Ki67 (black, H), and Bcl6 (dark, J) but not Bcl2 (dark, L). CD138+ plasma cells (dark grey) are exclusively detected in the stroma reaction (N) but never in Ti-BALT (data not shown). Original magnification: (A-D, K-L), x100; (E-J, M-N), x200. Abbreviation: T, Tumor nest.

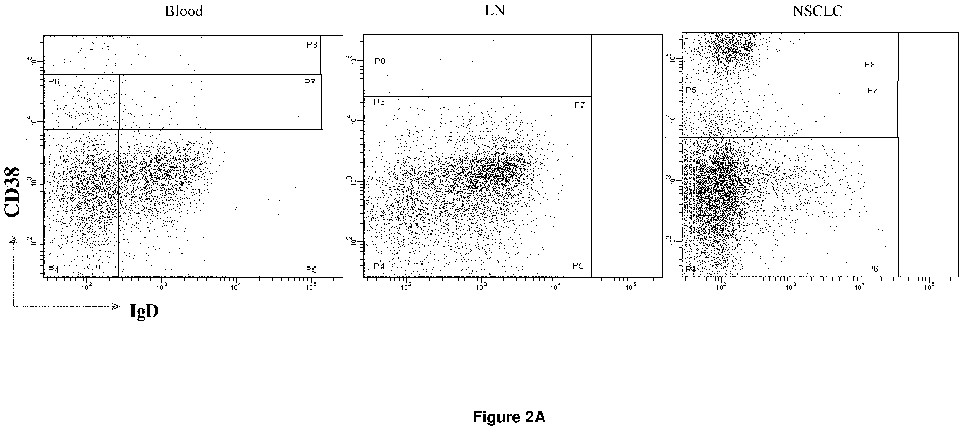

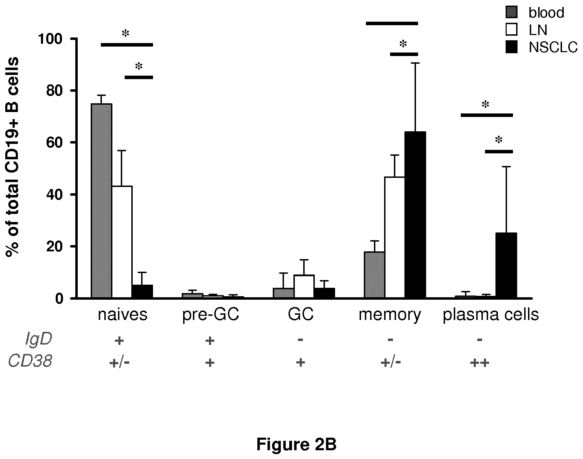

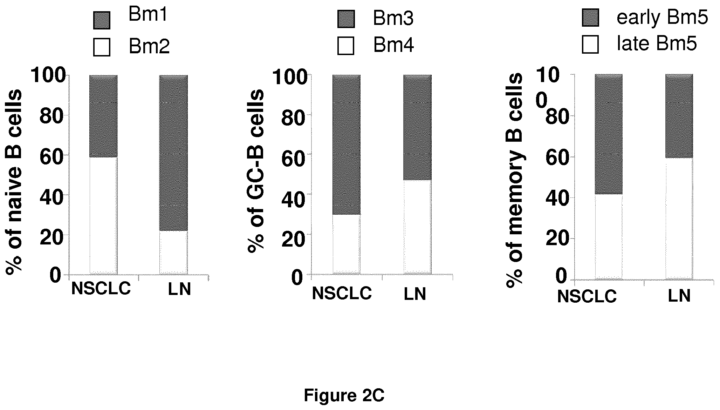

**Figure 2****. Comparison of the subsets of B cells infiltrating lung tumors, conventional secondary lymphoid organs and peripheral blood**
   Flow cytometry analysis of CD19+ CD14- B cell subsets on lung tumors (n=7), lymph nodes (n=3) and peripheral blood (n=5). Representative dot plots (A) and means (B) of B cell subsets based on the expression of IgD and CD38 on the 3 localizations. Each bar represents a mean +/- SD of different samples. Statistical significance of B cell subsets between sites was calculated by Mann-Whitney test. *, *P*<0.05. (C) Comparison of the ratio of the different differentiation stages of naive B cells (CD23- CD27- CD38- Bm1 and CD23+ CD27- CD38- Bm2, lelft panel), germinal centre B cells (CD23- CD27-CD77+ CD38+ Bm3 and CD23- CD27- CD77- CD38+ Bm4, centre panel) and memory B cells (CD23- CD27+ CD38+ early Bm5 and CD23- CD27+ CD38- late Bm5, right panel). Abbreviations: pre-GC, pre-germinal centre; GC, germinal centre.

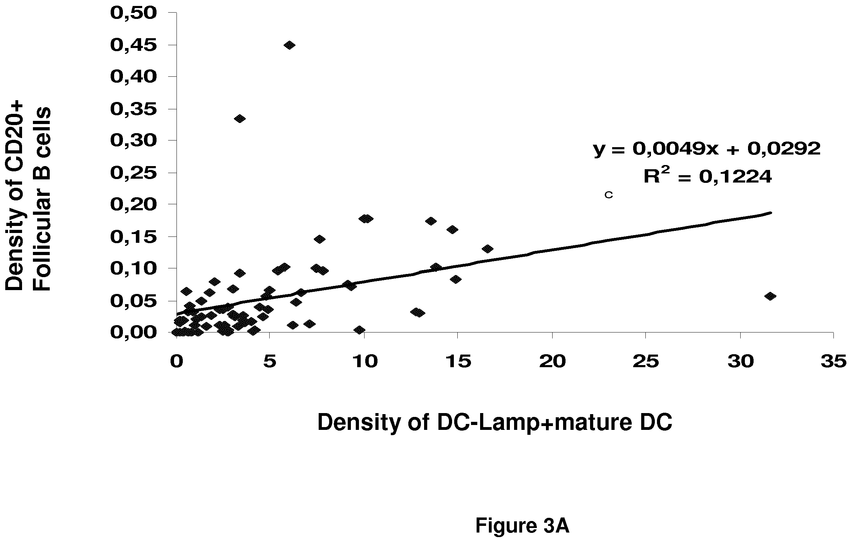

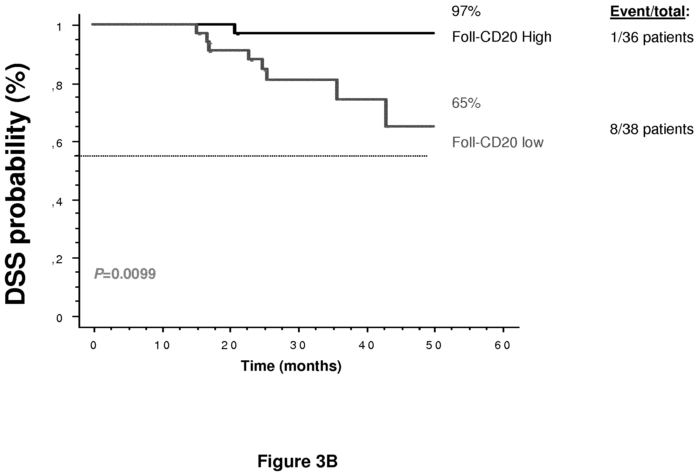

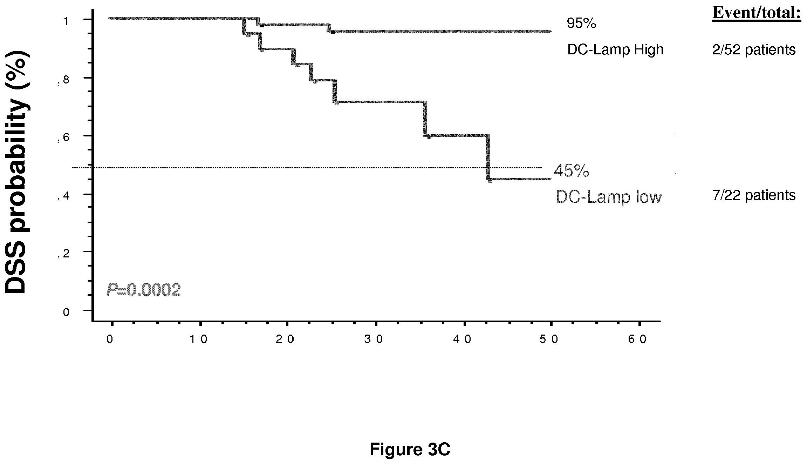

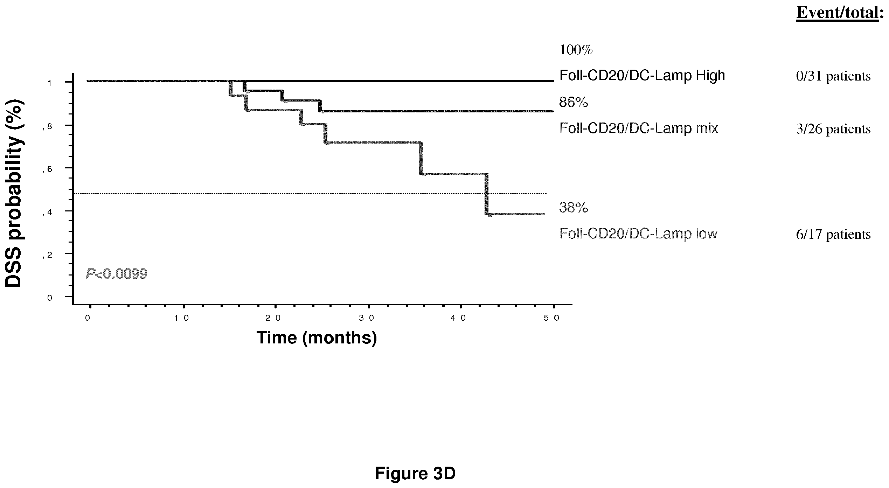

**Figure 3****. Prognostic value of Ti-BALT B cells in NSCLC patients**
   (A) Correlation between the density of DC-Lamp+ mature DC and the density of follicular CD20+ B cells, both populations located in Ti-BALT. Kaplan-Meier curves of disease-specific survival for 74 patients with early-stage NSCLC according to the density of CD20+ follicular B cells (B), the density of DC-Lamp+ mature DC (C), and the density of both cell populations (D). *P* value was determined using the log-rank test. Abbreviation: DSS, Disease-Specific Survival.

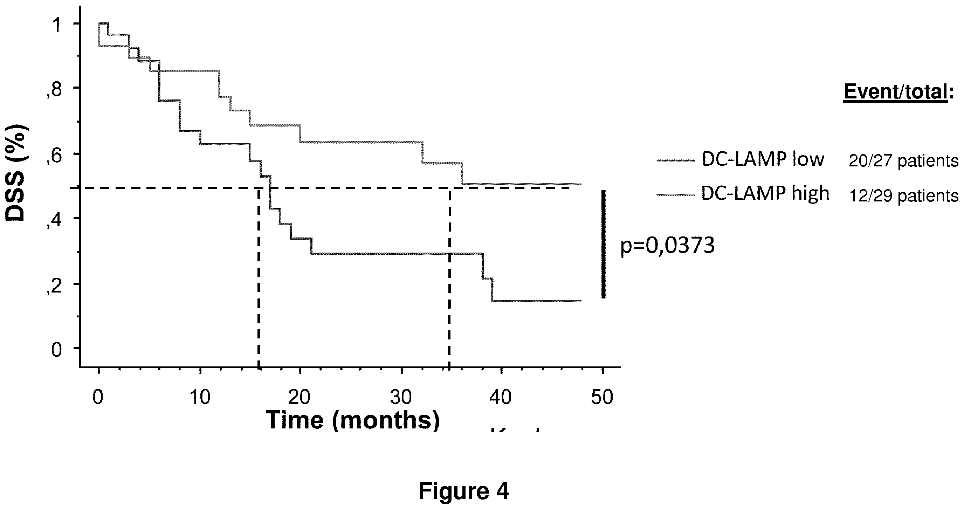

**Figure 4****. Prognostic value of mature DC in late-stage NSCLC patients who received neo-adjuvant chemotherapy.**
   Kaplan-Meier curves of disease-specific survival of 56 patients according to the density of tumor-infiltrating DC-Lamp+ mature DC. Significant differences between the two groups of patients were evaluated using the log-rank test. The median DSS was 17 months for the patients with DC-Lamp low tumors, whereas it was 36 months for the patients characterized as having DC-Lamp High tumors. P value significant when <0.05.

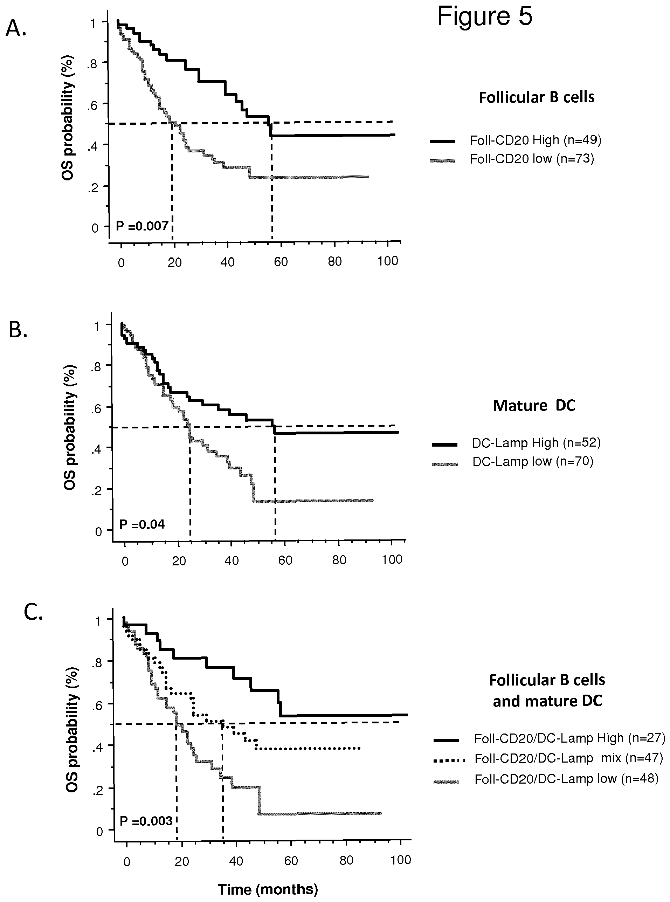

**Figure 5****: Prognostic value of tumor-infiltrating follicular B cells and/or mature DC in late-stage NSCLC patients who received neo-adjuvant chemotherapy.**
   Kaplan-Meier curves of overall survival of 122 patients with advanced-stage of NSCLC and treated by neo-adjuvant chemotherapy according to the presence of a high or low density of tumor-infiltrating (A) CD20+ follicular B cells, (B) DC-Lamp+ mature DC, or (C) both CD20+ follicular B cells and DC-Lamp+ mature DC. Significant differences between the groups of patients were evaluated using the Log-rank test. P value significant when <0.05. Abbreviation: OS, Overall Survival.

### TABLES

**Table 1. Clinical, histological and immunological parameters in patients with "Foll-B cells low" versus "Foll-B cells High" tumors**

| Pathologic staging and histologic types of lung cancer were determined according to the TNM staging system (Piemonte. NY, Wiley-Liss, 2002) and to the histologic classification of the WHO (Brambilla et al., Eur Respir. J., 2001), respectively. Abbreviations: NSCLC, Non-Small Cell Lung Cancer; ADC, Adenocarcinoma; SCC, Squamous Cell Carcinoma; pTNM, pathologic TNM. All parameters were evaluated among 74 early-stage NSCLC. P-values were obtained using the Fisher's and the Bonferroni-Dunn exact tests. Abbreviation: SEM, Standard Error of Measurements. | | | | | |
|---|---|---|---|---|---|
| | **Foll-B cells low** | | **Foll-B cells High** | | **Significance** (P value) |
| | No. | % | No. | % | |
| **Density of follicular CD20+ B cells** | | | | | **<0.0001** |
| mean±SEM | 0.011 ± 0.002 | - | 0.097 ± 0.014 | - | |
| range | 0.000-0.029 | | 0.031-0.442 | | |
| **Gender** | | | | | 0.0553 |
| male / female | 34/4 | 89/11 | 26/10 | 72/28 | |
| **Age** | | | | | 0.9665 |
| mean (years) ± SEM | 67 ± 1 | - | 67 ± 2 | - | |
| range | 49-83 | | 43-81 | | |
| **Smoking history** | | | | | 0,8374 |
| current/never smokers | 35/2 | 95/5 | 31/5 | 86/14 | |
| pack-years (years) ± SEM | 45 ± 4 | | 45 ± 5 | | |
| range | 0-100 | | 0-100 | | |
| **Histological type** | | | | | 0.8560 |
| ADC | 24 | 63 | 22 | 61 | |
| SCC | 14 | 37 | 14 | 39 | |
| **Tumor differentiation** | | | | | 0.0819 |
| well | 19 | 50 | 9 | 25 | |
| intermediate | 10 | 26 | 12 | 33 | |
| poorly | 9 | 24 | 15 | 42 | |
| **pTNM stage** | | | | | 0.3623 |
| pT1N0M0 | 27 | 71 | 21 | 58 | |
| pT2N0M0 | 7 | 18 | 7 | 20 | |
| pT1N1M0 | 4 | 11 | 8 | 22 | |
| **% fibrosis** | | | | | 0.1877 |
| mean±SEM | 22 ± 3 | - | 28 ± 3 | - | |
| range | 0-75 | | 0-85 | | |
| **% necrosis** | | | | | 0.6888 |
| mean±SEM | 13 ± 3 | - | 11 ± 3 | - | |
| range | 1-75 | | 1-50 | | |
| **% Ki67+ tumor cells** | | | | | 0.8895 |
| mean±SEM | 36 ± 4 | - | 36 ± 4 | - | |
| range | 1-80 | | 2-80 | | |
| **Density of DC-Lamp+ mature DC** | | | | | **0.0002** |
| mean±SEM | 2.652 ± 0.444 | - | 7.049 ± 1.034 | - | |
| range | 0-12.935 | | 0.500-31.667 | | |
| **Density of CD3+ T cells (centre of tumor)** | | | | | 0.2067 |
| mean±SEM | 1.579 ± 0.184 | - | 1.931 ± 0.206 | - | |
| range | 0.000-5.000 | | 0.000-5.000 | | |
| **Density of CD3+ T cells (invasive margin)** | | | | | **<0.0001** |
| mean±SEM | 1.487 ± 0.112 | - | 2.542 ± 0.172 | - | |
| range | 0.000-3.000 | | 0.500-5.000 | | |

**Table 2. Clinical and pathological features of NSCLC patients included in the retrospective study**

| Pathologic staging and histologic types of lung cancer were determined according to the TNM staging system (Piemonte. NY, Wiley-Liss, 2002) and to the histologic classification of the WHO (Brambilla et al., Eur Respir. J., 2001), respectively. Abbreviations: NSCLC, Non-Small Cell Lung Cancer; ADC, Adenocarcinoma; SCC, Squamous Cell Carcinoma; pTNM, pathologic TNM. | | |
|---|---|---|
| | Total | |
| | No. | % |
| **gender** | | |
| male / female | 60/14 | *81*/*19* |
| **age** | | |
| mean (years) ± SEM | 64±1 | |
| range | 41-79 | |
| **smoking history** | | |
| current/never smokers | 67/7 | *91*/*9* |
| pack-years (years) ± SEM | 45±3 | |
| range | 0-100 | |
| **vital status of patients** | | |
| alive | 54 | *73* |
| disease-free | 51 | |
| in relapse | 3 | |
| dead | 20 | *27* |
| from metastasis of NSCLC | 9 | |
| from other causes | 11 | |
| **histological type** | | |
| ADC | 46 | *62* |
| SCC | 28 | *38* |
| **pTNM stage** | | |
| pT1N0M0 | 48 | *65* |
| pT2N0M0 | 14 | *19* |
| pT1N1M0 | 12 | *16* |
| **tumor differentiation** | | |
| well | 28 | *38* |
| intermediate | 22 | *30* |
| poorly | 24 | *32* |
| **% fibrosis** | | |
| mean ± SEM | 25±2 | |
| range | 0-85 | |
| **% necrosis** | | |
| mean ± SEM | 12±2 | |
| range | 1-75 | |
| **% Ki67⁺ tumor cells*** | | |
| mean ± SEM | 36±3 | |
| range | 1-80 | |

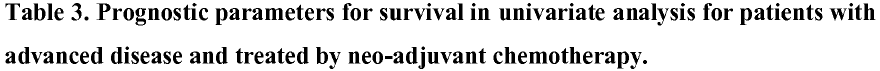

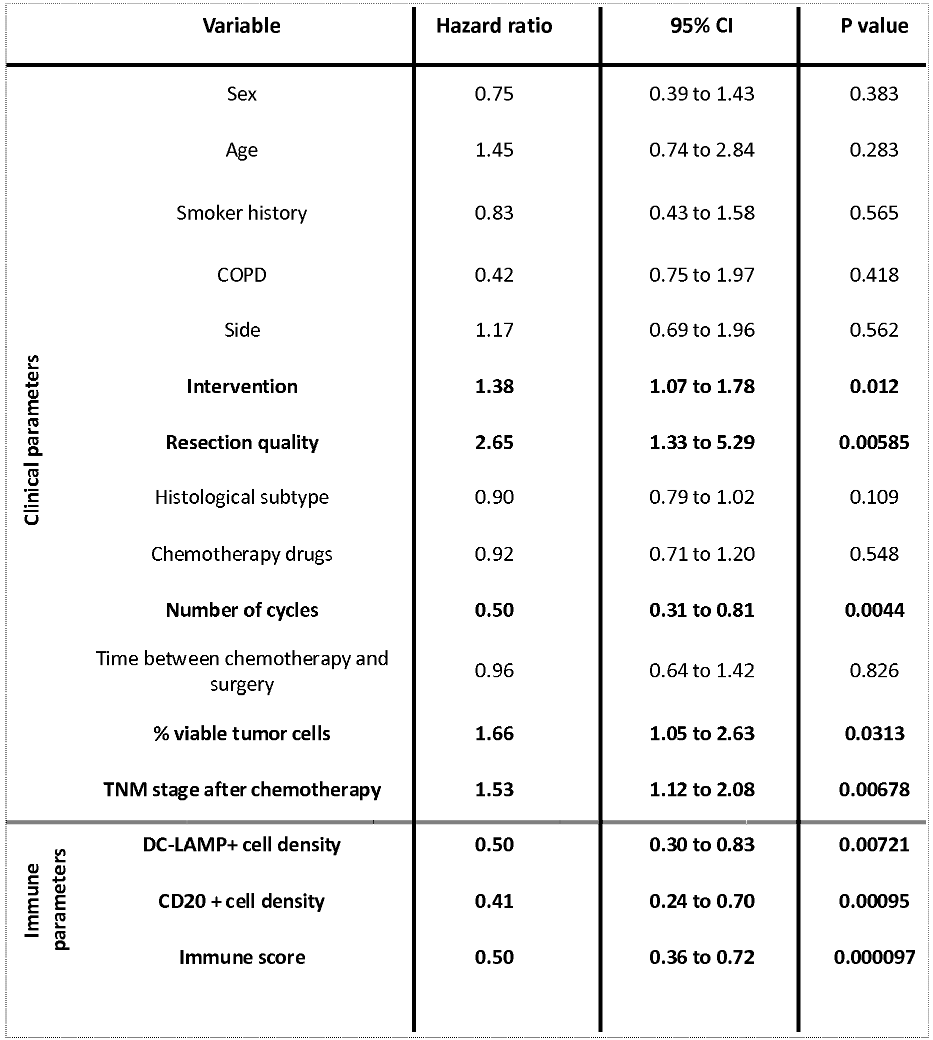

### EXAMPLES

In the following description, all molecular biology experiments for which no detailed protocol is given are performed according to standard protocol.

### Abbreviations

ADC, adenocarcinoma; BALT, Bronchus-Associated Lymphoid Tissue; DC, Dendritic Cell; NSCLC, Non-Small-Cell Lung Cancer; SCC, Squamous-Cell Carcinoma; TIL, Tumor-Infiltrating Lymphocyte.

### Example 1

### SUMMARY

The aim of the present study was to determine whether a protective humoral immune response takes place within Ti-BALT.

Here, we studied B-cell differentiation and migration to Ti-BALT by using complementary approaches (immunohistochemistry, flow cytometry, laser-capture micro-dissection, PCR low density array) on a series of 104 NSCLC patients. We have shown that B cell follicles of Ti-BALT present the same cellular composition and organization as in canonical secondary lymphoid organs. All stages of differentiation could be detected among tumor-infiltrating B cells. Interestingly, the major B cell compartments were compartments comprising effector cells (memory B cells and plasma cells). We have also shown that the somatic mutation and isotype switching machineries are activated in B cell follicles of Ti-BALT in accordance with the presence of Bm3 and Bm4 cells. We have also studied B cell recruitment to Ti-BALT in order to identify chemoattractants orchestrating this migration. Interestingly, intra-tumoral PNAd+ high endothelial venules were exclusively associated with Ti-BALT. The PNAd+ ligand, CD62L, was selectively detected on most Ti-BALT lymphocytes including all B cells but not on germinal center B cells, as reported for conventional secondary lymphoid organs. The analysis of the chemokine receptor profiles indicated that CXCR5 is expressed by naive B cells, which parallels the expression of its unique ligand, CXCL13 in the B-cell areas of Ti-BALT. Finally, CXCR5 expression decreased on fully differentiated B cells, a known key regulatory process that allows effector cells to leave the germinal centre, as previously described in lymph nodes.

Finally, we demonstrated that the density of Ti-BALT B cells is correlated with a long-term survival for lung cancer patients.

All together, these data indicate that Ti-BALT present strong similarity with canonical lymphoid organ, a site specialized in the induction and memory establishment of adaptive immune responses. Thus, Ti-BALT represents an active site for the initiation of a protective humoral immunity. The quantification of follicular B-cells would allow the identification of high-risk patients.

### Materials and methods

### Patients

Fresh and paraffin-embedded lung tumor samples were obtained from NSCLC patients undergoing surgery at Institut Mutualiste Montsouris, Hotel Dieu, Tenon and European Georges Pompidou Hospitals (Paris, France). Pre-operative evaluation of patients included lung, brain, and adrenal CT scan and liver ultrasound echography. They all underwent complete surgical resection of their tumors, including multilevel lymph node sampling or lymphadenectomy, but none received pre-operative chemotherapy or radiotherapy. Patients with an Eastern Cooperative Oncology Group performance status (Finkelstein et al., JCO, 1988) ≤ 1 were eligible. For the retrospective study, paraffin-embedded tumor biopsies with representative areas of tumor and adjacent lung parenchyma were retrieved from 74 successive patients diagnosed between 1998 and 2002 with early-stage NSCLC (Mountain, Cancer Chest, 1997). The main clinical and pathological features of the patients for the retrospective study are presented in Table 2. Patients with mixed histologic features, a T3 tumor, or pleural invasion were ineligible. At the completion of the study, the minimal clinical follow-up was 48 months for the last patient included in the cohort. Non-tumoral lymph nodes were obtained after surgery from patients suffering from cardiac diseases. Peripheral blood was obtained from healthy volunteers at the "Centre National de la Transfusion Sanguine" (Paris, France). The protocol was approved by local ethic and human investigations committee (n° 2008-133), and by the Assistance Publique-Hopitaux de Paris (AP-HP), in application with the article L.1121-1 of French law. A written informed consent was obtained from the patients prior to inclusion in the study.

### Immunohistochemistry

Serial 5µm tissue sections of paraffin-embedded lung tumors were deparaffinized, rehydrated, and pretreated in appropriate buffer for antigen retrieval. Then, the sections were incubated with 5% human serum for 30 min before adding the appropriate antibodies or isotype controls. Enzymatic activity was revealed as described previously (MCD, JCO, 2008). When necessary, sections were counterstained with hematoxylin. Images were acquired using a Nikon Eclipse 80i microscope (Nikon, Champigny-sur-Marne, France) operated with Nikon NIS Elements BR software.

### Method for cell quantification

The cell quantification was measured quantitatively in the tumoral areas of the entire tissue section (original magnification: x100) using the Nikon Eclipse 80i microscope and operated with Nikon NIS Elements BR software. The density of follicular B cells of Ti-BALT was expressed as a surface of follicular CD20+ B cells per tumor intermediate power field (IPF) with SEM calculated. The number of DC-Lamp⁺ mature DC was lower than the number of cells described above allowing us to realize a quantitative counting (mean DC per tumor IPF with SEM calculated). According to the standard evaluation by pathologists, the necrosis and fibrosis were counted as the percentage of the positive areas among the whole tumor mass section.

### Enrichment of tumor-infiltrating lymphocytes

Fresh lung tumor specimens were mechanically dissociated and incubated in a non-enzymatic solution (Cell Recovery solution, BD Biosciences, Le Pont-de-Claix, France) for 1h at 4°C. The cell suspension was then filtrated through a 70µm filter (BD Biosciences), and the mononuclear cells were isolated by centrifugation over Ficoll Hypaque.

### Flow cytometry

Multiple stainings were performed using antibodies against B cell markers. Briefly, after saturation with 2% human serum, mononuclear cells were incubated with the primary antibodies or appropriate isotype controls for 30 min at +4°C in the dark. Then, cells were washed and fixed in 1% formaldehyde before the analysis on a LSRII cytometer (BD Biosciences). Flow cytometry data were analyzed with the Diva software (BD Biosciences).

### Ex vivo culture of tumor-infiltrating B cells

Total tumor-infiltrating B cells were isolated from mononuclear cells through a positive selection. Briefly, cells were incubated in presence of anti-CD19 microbeads (Miltenyi Biotec), and then loaded onto a Macs columns (Miltenyi Biotec). Freshly isolated B cells were cultured in presence of Pansorbin (Staphylococcus aureus cells extract, Calbiochem) or murine CD40 ligand transfected fibroblastic cell line (CD40-L L cells were kindly provided by Schering-Plough, Laboratory for Immunological Research, Dardilly, France). The supernatant was recovered every 3 days until day 9, and cryopreserved.

### Statistical analysis

Variables taken into account for statistical analysis included clinical (age, sex, smoking, tumor relapse, and vital status), histopathological (histology, pTNM, tumor differentiation, localization of the primary tumor, necrosis, fibrosis, proliferation of the tumor) and immunological parameters (see markers described above). To perform univariate analysis, groups of patients were defined according to the bimodal distribution of the density of positive cells which appointed the following cut-offs. (follicular CD20+ B cells: 0.029 mm²/ tumor IPF, DC-Lamp: 1.65 mean cells/tumor IPF). Chi-square test with Yates correction and ANOVA test (post-hoc tests with Fisher and Bonferonni methods) were used for univariate analysis. Disease-specific survival (DSS) curve were estimated by Kaplan-Meier method and the significance of differences between groups of patients was evaluated by the Logrank test. An event affecting the OS was defined as death from any cause, DSS as death from NSCLC and DFS as relapse of the primary tumor. Statistical analysis was performed using StatView software. A *P* value < 0.05 was considered statistically significant.

### Results

### 1 - Ti-BALT contain the same contingent of immune cells as that observed in secondary lymphoid organs

In order to study the role of the B-cell compartment of Ti-BALT, we first characterized by immunohistochemistry B cells and other immune cells in which they will be in contact in these tertiary lymphoid structures. As described in conventional lymphoid organs, we observed that CD3+ T cells and DC-Lamp+ mature DC cluster to form the T-cell rich areas whereas most CD20+ B cells segregate into B follicles. Within the B-cell areas, different non-B cells were present. We detected few CD3+ T cells which probably represent follicular helper T cells, follicular dendritic cells which are organized into a network and a specialized population of CD68+ macrophages also called tangible-body macrophages.

In conclusion, the segregation of immune cells in Ti-BALT is the same as that observed in secondary lymphoid organs, suggesting that immune responses may take place within ectopic lymphoid structures in human lung cancer.

### 2 - The B-cell areas of Ti-BALT have features of an ongoing humoral immune response

The segregation of T and B-cell areas is a mandatory for the development of both high-affinity class-switched antibodies and memory humoral immune response. Thus, we assessed the stage of B-cell differentiation within the lung tumors, and compared it to secondary lymphoid organs. By immunohistochemistry, we first characterized B-cell subsets according to the Bm classification proposed by Dr. D. Capra (Pascual et al., J. Exp. Med., 1994). As observed in the secondary follicles of lymph nodes (fig. 1A), we shown an accumulation of IgD+ naive B cells in a restricted areas called the mantle (fig. 1B). This mantle surrounded a germinal centre (GC) as defined by the presence of CD23+ cells which comprised a network of follicular dendritic cells and Bm2 naive B cells (fig. 1C-D). GC-B cells were also characterized by the expression of AID (fig 1E-F), the enzyme critical for the somatic hypermutation, class switch recombination and gene conversion of immunoglobulin (Ig) genes. In a similar manner, GC-B cells were positive for the proliferation marker Ki67 (fig. 1G-H) and Bcl6 (fig. 1I-J), but did not express the anti-apoptotic protein Bcl2 (fig. 1K-L) in Ti-BALT and lymph nodes. Based on the expression of CD138, no plasma cell (PC) was detected in Ti-BALT B follicles (data not shown), in accordance with the situation seen in lymph nodes (fig 1M). However, CD138+ PC were observed in the stroma and the fibrosis of the tumor (fig. 1N).

We next further compared the proportion of intra-tumoral B cell subsets to lymph nodes as well as peripheral blood by flow cytometry. According to the expression of IgD and CD38 among total CD19+ cells, the distribution of the five B cell subsets was completely distinct in NSCLC compared to lymph nodes and blood (Fig. 2A). Blood and LN-B cells were mainly of naive and memory phenotypes (IgD+ CD38low naive B cells, IgD- CD38low memory B cells, respectively). In contrast to blood and lymph nodes, every stage of B cell differentiation was detected in NSCLC. We shown that the percentage of memory B cells and PC was statistically higher, and naive B cells lower in NSCLC compared to the two other sites (Fig. 2B; 18%, 48%, and 62% of memory cells ; 1%, 0.5% and 28% of PC; 75%, 42% and 4% of naive B cells in blood, lymph nodes and NSCLC). The differential expression of additional B cell markers (CD23, CD27, CD77) allowed us to study the distribution of subsets of naive (Bm1 and Bm2), GC (Bm3 and Bm4) and memory (early Bm5 and late Bm5) B cells in NSCLC and lymph nodes. As shown in Fig. 2C, the main difference between the 2 sites was the ration of Bm1/Bm2 which was in favor of Bm1 in lung tumor. The other ratios analyzed (Bm3/Bm4 and early Bm5/late Bm5) were not statistically different in the 2 anatomic sites.

All together, these data demonstrate that the differentiation stage of tumor-infiltrating B cells is in accordance with their in situ localization, i.e. in or out of ectopic lymphoid structures. Early differentiated B cells are organized into B follicles of Ti-BALT where the somatic mutation and isotype switching machineries are activated, suggesting that Ti-BALT may be an active site for the generation of memory B cells and antibody-secreting cells.

### 3 - Density of follicular B cells is correlated with long-term survival, and its prognostic value is enhanced when associated with the density of mature DC

The presence of reactive B follicles in some lung tumors prompted us to investigate their immunological function. As we have shown that the density of mature DC was associated with a favourable clinical outcome (Dieu-Nosjean et al., J. Clin. Oncol., 2008), we investigated whether the densities of follicular B cells and mature DC were correlated to each other as well as their prognostic value. Figure 3A shows that, even if the global increase of the density of follicular B cells was associated with a global increase of the density of mature DC, these two parameters were not statistically related to each other (R²=0.1224) suggesting their reciprocal independence. By univariate analysis, we next investigated the prognostic value of follicular B cells alone or in combination with mature DC. The four-year disease-specific survival rates were 97% among patients with high density of follicular B cells (Foll-CD20 High), and 65% among patients with low density of follicular B cells (Foll-CD20 low) (Fig. 3B). Thus, the patients with Foll-CD20 High have a longer survival then patients with Foll-CD20 low (P=0.0099) demonstrating that the number of follicular B cells was associated with a favorable prognosis. There were no distinguishable clinical (sex, age, smoking history), tumor (tumor differentiation, pTNM staging, fibrosis, necrosis, and proliferating tumor cells), or histologic characteristics between the patients with Foll-CD20 High *versus* Foll-CD20 low tumors (Table 1). A similar result was obtained for the density of DC-Lamp+ mature DC (Fig. 3C) indicating that both professional antigen-presenting cells were predictive for survival. We next tested whether the patients with "DC-Lamp High" tumors and patients with "Foll-CD20 High" tumors were the same, and *vise versa* for patients with "DC-Lamp low" tumors and "Foll-CD20 low" tumors. Among the 74 patients, 31 patients (42% of patients) belonged to both groups of "DC-Lamp High" tumors and "Foll-CD20 High" tumors, 17 (23% of patients) belonged to both groups of "DC-Lamp low" tumors and "Foll-CD20 low" tumors, and 26 (35% of patients) were mixed. Among this mix group called "Foll-CD20/DC-Lamp mix", 21 patients were characterized as having "DC-Lamp High" tumors and "Foll-CD20 low" tumors, and 5 patients with "DC-Lamp low" tumors and "Foll-CD20 High" tumors. The presence of this non-overlapping group is in favour of an independence between these 2 parameters. Due to the limited number of patients within each sub-group of the mix group, we decided to keep the 26 patients within a unique group. The Kaplan-Meier curves indicated that 100% of patients with "Foll-CD20/DC-Lamp high" were alive (none event among the 31 patients) after a follow-up of 48 months (Fig. 3D). We found that patients with low density of both intra-tumoral mature DC and follicular B cells ("Foll-CD20/DC-Lamp low" group) had a very poor prognosis with 38% of surviving patients after 48 months (6 events out of 17 patients). The survival curves of patients with "Foll-CD20/DC-Lamp mix" tumors were between these two curves with 86% of alive patients (3 events out of 26 patients). The median disease-specific survival was only reached for the patients with "Foll-CD20/DC-Lamp low" tumors (42 months, P<0.0099).

In conclusion, we demonstrated that the density of tumor-infiltrating follicular B cells is highly predictive of disease-specific survival in early-stage NSCLC. Compared to the density of each cell type, the combination of both mature DC and follicular B cells allows the identification of a group of patients without any event and a group with most events.

### Example 2

### 1 - Prognostic value of follicular B cells and of mature dendritic cells in patients treated by neo-adjuvant chemotherapy

The five-year survival of patients with early-stage NSCLC is 70%, and drops to 15% for late-stage metastastic NSCLC. Studies have shown that two-drug combinations are more efficacious than single-agent treatment (Schiller et al., 2000). Presently, patients with advanced NSCLC receive a neo-adjuvant polychemotherapy (cisplatin plus gemcitabine or carboplatin plus paclitaxel) in many North American and European hospitals (Bunn et al., 2002 ; Rosell et al., 2002). Now, more and more patients with early-stage lung cancer also receive neo-adjuvant chemotherapy. The response rate of two-drug combinations is between 20 to 30% in advanced NSCLC. Recent reports indicate that cytotoxic drugs are not only targeting tumor cells, but can indirectly promote tumor control by facilitating the development of an immune response within the tumor microenvironment (reviewed in Zitvogel et al., 2008).

Lung cancer contains tumor cells as well as stroma components: the vasculature, connective tissue and immune infiltrating cells. Among immune cell infiltrate, some of them are professionals for the presentation of processed antigens, like DC, B cells and macrophages. Recently, we have shown that the density of different DC subsets (epithelial Langerhans cells, stromal interstitial DC and mature DC) was associated with a favorable outcome in NSCLC patients (Dieu-Nosjean et al., 2008; Fridman et al., 2011). Now, we show that the density of mature DC is still correlated with a longer-term survival for advanced NSCLC patients treated by neo-adjuvant chemotherapy (n=56 patients, P=0.0373, see Figure 4).
As Ti-BALT are present in patients treated by chemotherapy, the density of B cells, another antigen-presenting cell population, can be associated with the outcome of patients treated by chemotherapy. The density of B cells may also be impacted by the type of the two-drug combinations. Thus, the B-cell marker will be correlated to clinical response to treatment. This biomarker can be used to discriminate between patients who will respond and patients who will not respond to the treatment, and thus allowing a more rational and targeted therapy design.

### Example 3

Here, we evaluated the prognostic value of either follicular B cells, mature DC, or the combination of both types of immune cells in a retrospective study of 122 patients with advanced-stage of NSCLC and treated by neo-adjuvant chemotherapy. We demonstrated that the density of each immune parameter was correlated with a favorable outcome. The Kaplan-Meier curves indicated that the density of CD20+ follicular B cells was associated with longer overall survival (OS, P=0.007) (Fig. 5A). The median OS was 55 months for the patients characterized as having Foll-CD20 High tumors whereas the median OS was 18 months for patients with Foll-CD20 low tumors. The density of DC-Lamp+ mature DC was also correlated with a better clinical outcome (Fig. 5B, P=0.04). The median OS was 55 months for the patients with DC-Lamp High tumors whereas the median OS was 24 months for patients with DC-Lamp low tumors.

As Foll-CD20 and DC-Lamp positively influence the patient survival, we stratified the patients into 3 groups according to the high/low densities of each marker (Foll-CD20/DC-Lamp High, Foll-CD20/DC-Lamp mix, and Foll-CD20/DC-Lamp low). Patients with high density of both types of immune populations were at low risk of death (median OS was not reached) (Fig. 5C, P=0.003). Patients with low densities of both follicular B cells and mature DC were at very high risk of death (median OS was 18 months), demonstrating that the combination of both immune markers allows the identification of a subgroup of patients with a very poor outcome despite chemotherapy. Patients with "Foll-CD20/DC-Lamp mix" tumors were at intermediate risk of death (median OS was 34 months).

These data were in accordance with univariate analyses (Table 3) showing that the density of each immune cell type is highly associated with the survival of patients treated by chemotherapy (Foll-CD20 with a Hazard Ratio (HR) =0.41 and P=0.00095; DC-Lamp with a HR=0.50 and P=0.00721). More interestingly, the combination of both biomarkers was the better predictor for survival (HR=0.50, P=0.000097) compared to standard clinical parameters.

All together, we have demonstrated that the combination of CD20 and DC-Lamp markers is able to discriminate patients with very high risk of death among advanced-stage NSCLC patients treated by neo-adjuvant chemotherapy.

### References

Throughout this application, various references describe the state of the art to which this invention pertains.
Brambilla E, Travis WD, Colby TV, et al: The new World Health Organization classification of lung tumours. Eur Respir J. 2001 ; 18:1059-1068.
Bunn PA Jr: Treatment of advanced non-small-cell lung cancer with two-drug combinations. J. Clin. Oncol. 2002; 20:3565-3567.
Dieu-Nosjean MC, Antoine M, Danel C, Heudes D, Wislez M, Poulot V, Rabbe N, Laurans L, Tartour E, de Chaisemartin L, Lebecque S, Fridman WH, Cadranel J. Long-term survival for patients with non-small-cell lung cancer with intratumoral lymphoid structures. J Clin Oncol. 2008; 26(27):4410-7.
Finkelstein DM, Cassileth BR, Bonomi PD, et al.: A pilot study of the Functional Living Index-Cancer (FLIC) Scale for the assessment of quality of life for metastatic lung cancer patients: An Eastern Cooperative Oncology Group study. Am J Clin Oncol. 1988 ;11:630-633.
Gobert M, Treilleux I, Bendriss-Vermare N, Bachelot T, Goddard-Leon S, Arfi V, Biota C, Doffin AC, Durand I, Olive D, Perez S, Pasqual N, Faure C, Ray-Coquard I, Puisieux A, Caux C, Blay JY, Ménétrier-Caux C. Regulatory T cells recruited through CCL22/CCR4 are selectively activated in lymphoid infiltrates surrounding primary breast tumors and lead to an adverse clinical outcome. Cancer Res. 2009; 69(5):2000-9.
Mountain CF: Revisions in the International System for Staging Lung Cancer. Chest. 1997 ; 111:1710-1717.
Pascual V, Liu YJ, Magalski A, de Bouteiller O, Banchereau J, Capra JD. Analysis of somatic mutation in five B cell subsets of human tonsil. J Exp Med. 1994 ; 180(1):329-39.
Piemonte M: International Union Against Cancer: TNM Classification of Malignant Tumors (ed 6). New York, NY, Wiley-Liss, 2002.
Rosell R, Gatzemeier U, Betticher DC, et al: Phase III randomised trial comparing paclitaxel/carboplatin with paclitaxel/cisplatin in patients with advanced non-small-cell lung cancer: A cooperative multinational trial. Ann. Oncol. 2002; 13:1539-1549.
Sautes-Fridman C, Cherfils-Vicini J, Damotte D, Fisson S, Fridman WH, Cremer I, Dieu-Nosjean MC. Tumor microenvironment is multifaceted. Cancer Metastasis Rev. 2011 ; 30(1):13-25.
Schiller JH, Harrington D, Belani C, et al: Comparison of four chemotherapy regimens for advanced non small cell lung cancer. N. Engl. J. Med., 2000; 346:92-98.
Sobin L, Wittekind. TNM classification of malignant tumors, Wiley-Liss, ed. 6, New York, 2002.
Zitvogel L, Apetoh L, Ghiringhelli F, Kroemer G. Immunological aspects of cancer chemotherapy. Nature Rev. Immunol. 2008 ;8(1):59-73.

## Claims

1. An *in vitro* method for the prognosis of survival time of a patient suffering from a solid cancer with tumor-induced lymphoid structures, comprising the following steps:
a) quantifying, in a tumor-induced lymphoid structure , the cell density of follicular B cells, and
b) comparing the cell density of follicular B cells value obtained at step a) with a predetermined reference value; and
c) providing a favorable prognosis of survival time for said patient when the cell density of follicular B cells is higher than said predetermined reference value, or
providing a poor prognosis of survival time for said patient when the cell density of follicular B cells is lower than said predetermined reference value.

2. The method of claim 1 wherein the survival is the disease-specific survival (DSS), the disease-free survival (DFS) or the overall survival (OS).

3. The method according to claim 1 or 2 further comprising the steps of:
a) quantifying on the whole tumor, the cell density of mature dendritic cells, and
b) comparing the cell density of mature dendritic cells value obtained at step a) with a predetermined reference value; and
c) providing a favorable prognosis of survival time for said patient when the cell densities of both the follicular B cells and of the mature dendritic cells are higher than said predetermined reference values,
providing a poor prognosis of survival time for said patient when the cell densities of both follicular B cells and of the mature dendritic cells are lower than said predetermined reference values, or
providing an intermediate prognosis of survival time for said patient when one value of the cell densities of follicular B cells and of the mature dendritic cells is lower than said predetermined reference value and the other value is higher than said other predetermined reference value.

4. The method of any of claims 1 to 3 wherein the solid cancer is selected from the group consisting of lung cancers, colorectal cancers and breast cancers.

5. The method of claim 4 wherein the solid cancer is a lung cancer.

6. The method of claim 4 wherein the solid cancer is a non-small cell lung cancer.

7. The method of any of claims 1 to 6 wherein the patient is a patient with an early stage of cancer.

8. The method of claim 7 wherein the patient is a patient with early stage of cancer who did not receive any neo-adjuvant or adjuvant therapy.

9. The method of any of claims 1 to 6 wherein the patient is a patient with advanced stage of cancer.

10. The method of claim 9 wherein, the patient is a patient with advanced stage of cancer who receives adjuvant therapy.

## Patentansprüche

1. Ein *in vitro*-Verfahren zur Prognose der Überlebenszeit eines Patienten, der an einem soliden Krebs mit Tumor-induzierten lymphoiden Strukturen leidet, umfassend die folgenden Schritte:
a) Quantifizieren der Zelldichte von follikulären B-Zellen in einer Tumor-induzierten lymphoiden Struktur, und
b) Vergleich des Werts der Zelldichte der follikulären B-Zellen, erhalten in Schritt a) mit einem vordefinierten Referenzwert; und
c) Bereitstellen einer günstigen Prognose für die Überlebenszeit dieses Patienten, wenn die Zelldichte der follikulären B-Zellen höher ist als der vorbestimmte Referenzwert oder Erstellen einer ungünstigen Prognose für die Überlebenszeit dieses Patienten, wenn die Zelldichte der follikulären B-Zellen niedriger ist als der vorbestimmte Referenzwert.

2. Verfahren nach Anspruch 1, wobei das Überleben das erkrankungsspezifische Überleben (DSS), das erkrankungsfreie Überleben (DFS) oder das Gesamtüberleben (OS) ist.

3. Verfahren nach Anspruch 1 oder 2, weiter umfassend die Schritte von:
a) Quantifizieren der Zelldichte von reifen dendritischen Zellen im Gesamttumor, und
b) Vergleich des Werts der Zelldichte von reifen dendritischen Zellen, erhalten aus Schritt a), mit einem vorbestimmten Referenzwert; und
c) Erstellen einer günstigen Prognose für die Überlebenszeit dieses Patienten, wenn die Zelldichten sowohl der follikulären B-Zellen als auch reifen dendritischen Zellen höher sind als die vorbestimmten Referenzwerte,
Erstellen einer ungünstigen Prognose für die Überlebenszeit dieses Patienten, wenn die Zelldichten sowohl der follikulären B-Zellen als auch der reifen dendritischen Zellen niedriger sind als die vorbestimmten Referenzwerte, oder
Erstellen einer intermediären Prognose für die Überlebenszeit dieses Patienten, wenn ein Wert der Zelldichten der follikulären B-Zellen und der reifen dendritischen Zellen niedriger ist als der vorbestimmte Referenzwert und der andere Wert höher ist als der andere vorbestimmte Referenzwert.

4. Verfahren nach einem der Ansprüche 1 bis 3, wobei der solide Krebs ausgewählt ist aus der Gruppe, bestehend aus Lungenkrebs-, Kolorektalkrebs- und Brustkrebserkrankungen.

5. Verfahren nach Anspruch 4, wobei der solide Krebs ein Lungenkrebs ist.

6. Verfahren nach Anspruch 4, wobei der solide Krebs ein nicht-kleinzelliger Lungenkrebs ist.

7. Verfahren nach einem der Ansprüche 1 bis 6, wobei der Patient ein Patient in einem Frühstadium von Krebs ist.

8. Verfahren nach Anspruch 7, wobei der Patient ein Patient mit einem Frühstadium von Krebs ist, der keine neo-adjuvante oder adjuvante Therapie erhalten hat.

9. Verfahren nach einem der Ansprüche 1 bis 6, wobei der Patient ein Patient in einem fortgeschrittenen Stadium von Krebs ist.

10. Verfahren nach Anspruch 9, wobei der Patient ein Patient mit einem fortgeschrittenen Stadium von Krebs ist, der eine adjuvante Therapie erhält.

## Revendications

1. Procédé *in vitro* de pronostic du temps de survie d'un patient souffrant d'un cancer solide avec des structures lymphoïdes induites par la tumeur, comprenant les étapes suivantes :
a) quantification, dans une structure lymphoïde induite par la tumeur, de la densité cellulaire de cellules B folliculaires, et
b) comparaison de la valeur de la densité cellulaire de cellules B folliculaires obtenue à l'étape a) avec une valeur de référence prédéterminée ; et
c) apport d'un pronostic de temps de survie favorable pour ledit patient lorsque la densité cellulaire de cellules B folliculaires est plus élevée que ladite valeur de référence prédéterminée, ou
apport d'un pronostic de temps de survie défavorable pour ledit patient lorsque la densité cellulaire de cellules B folliculaires est plus basse que ladite valeur de référence prédéterminée.

2. Procédé selon la revendication 1 dans lequel la survie est la survie spécifique à la maladie (DSS), la survie sans maladie (DFS) ou la survie globale (OS).

3. Procédé selon la revendication 1 ou 2 comprenant en outre les étapes de :
a) quantification sur la tumeur entière, de la densité cellulaire de cellules dendritiques matures, et
b) comparaison de la valeur de la densité cellulaire de cellules dendritiques matures obtenue à l'étape a) avec une valeur de référence prédéterminée ; et
c) apport d'un pronostic de temps de survie favorable pour ledit patient lorsque les densités cellulaires à la fois des cellules B folliculaires et des cellules dendritiques matures sont plus élevées que lesdites valeurs de référence prédéterminées,
apport d'un pronostic de temps de survie défavorable pour ledit patient lorsque les densités cellulaires à la fois des cellules B folliculaires et des cellules dendritiques matures sont plus basses que lesdites valeurs de référence prédéterminées, ou
apport d'un pronostic de temps de survie intermédiaire pour ledit patient lorsqu'une valeur des densités cellulaires de cellules B folliculaires et des cellules dendritiques matures est plus basse que ladite valeur de référence prédéterminée et que l'autre valeur est plus haute que ladite autre valeur de référence prédéterminée.

4. Procédé selon l'une quelconque des revendications 1 à 3, dans lequel le cancer solide est choisi dans le groupe consistant en les cancers du poumon, les cancers colorectaux et les cancers du sein.

5. Procédé selon la revendication 4, dans lequel le cancer solide est un cancer du poumon.

6. Procédé selon la revendication 4, dans lequel le cancer solide est un cancer du poumon non à petites cellules.

7. Procédé selon l'une quelconque des revendications 1 à 6, dans lequel le patient est un patient à un stade précoce du cancer.

8. Procédé selon la revendication 7, dans lequel le patient est un patient à un stade précoce du cancer qui n'a reçu aucun traitement néoadjuvant ou adjuvant.

9. Procédé selon l'une quelconque des revendications 1 à 6, dans lequel le patient est un patient à un stade avancé du cancer.

10. Procédé selon la revendication 9, dans lequel le patient est un patient à un stade avancé du cancer qui reçoit un traitement adjuvant.
